# EUROPEAN PATENT APPLICATION

(11) **EP 1 815 784 A1**
(43) Date of publication of application: **08.08.2007**
(21) Application number: 06009509.8
(22) Date of filing: 09.05.2006
(51) Int. Cl.: A61B 5/00

(54) **System with intelligent cable-less transducers for monitoring and analysing biosignals**

(30) Priority: 06.02.2006 EP 06002331
(71) Applicant: Amer, Mashhour Mustafa Moh'd Bani, Irbid (JO); Izraiq, Mahmoud Ibrahim Mahmoud, Amman 11121 (JO)
(72) Inventor: Amer, Mashhour Mustafa Moh'd Bani, Irbid (JO); Izraiq, Mahmoud Ibrahim Mahmoud, Amman 11121 (JO)
(74) Representative: Bittner, Thomas L.

(57) **Abstract**

A mobile wireless system for capturing human biosignals via cable-less button-like transducers placed on human skin or implanted inside the human body of the patient. The mobile system includes an Intelligent miniaturized cable-less transducer, a wireless mobile unit, a computing device (mobile phone handset, Personal Digital Assistant (PDA)), or Personal Computer (PC)), and Internet/ Intranet servers. The system may be used to monitor different bio-potentials (ECG, EEG, ERG, EMG, and EOG) and biosignals such as glucose, Phonocardiography and blood pressure. Captured information is presented to the physicians in appropriate medial presentation formats both numerically and graphically.

## Description

The present invention relates generally to biomedical devices and biosignal capturing, analysis, persistence, reporting, and remote access, and more particularly to dis-joining the physician from the patient allowing patient mobility and remoteness and cable-less data gathering and transmission system.

### Background of the invention

In traditional diagnosis methods, the patient must make physical presence to the medical center that would have the necessary instruments, which are usually expensive and not portable. This presents complex issues as there are many medical cases that require prompt acquisition of patients biosignals; for example in case of biopsy and arrhythmias, the time needed for the patient to be transferred to the hospital may very likely have the problem gone, and hence physicians will not be able to know exactly what happened, therefore they cannot make any medical decisions. Otherwise, the patient needs to be hospitalized in a care unit for as long as needed until that issue appears again under the monitoring instruments.

In conventional clinical monitoring and diagnostic instruments, such as in case of monitoring ECG, EEG, and Parkinson disease several electrodes are attached to a patient's body and then connected through wires to the ECG or EEG system. In an operating room and intensive care units, wires for different ECG and EEG leads and other equipments such as pulse oximeter and pressure meters may have to be connected between the patient's body and the monitoring system. This presents a particularly complex cable management problem for the attending physician or nurse. Considerable time can be consumed in disentangling the patient when they must be disconnected or transferred to another area of the hospital. On another hand, in the implantable artificial organs, wires need to exist between the implanted electronic device and the external monitoring device, causing severs health issues to the patient.

The mobile system for wireless measurement of biosignals comprises a miniaturized intelligent cable-less transducers and a mobile unit. Thus, by eliminating wires, the biosignals can be quickly and easily setup in any environment. The lack of wires greatly reduces the restriction on the subject's movement, which in turn leads to more accurate diagnoses. Patients can continuously be monitored in their every day life, and this gives possibilities for increased safety, and increased quality of life. Thus, there are better possibilities for managing chronic care, controlling health delivery costs, increasing patient quality of health services, and predicting and thus avoiding serious complications.

The miniaturized cable-less transducers are used to transmit the biosignals wirelessly to the mobile unit, which could be either worn by the patient or placed in his room or apartment, with obvious advantages for patient comfort and compliance. These transducers might be either cable-less electrodes that can be used for measurement of biopotentials, namely Electrocardiograph (ECG), Electroencephalograph (EEG), Electromyograph (EMG), Electroretinograph (ERG), or cable-less sensors for the measurement of biomedical signals such as blood pressure, temperature, respiratory parameters, blood glucose, blood gases, and electrolytes. Such cable-less transducers will greatly improve the use and applications of prosthetic devices and artificial organs especially the artificial pancreas and cochlear implants. These transducers integrate signal acquisition, signal conditioning, and signal transmission on the same miniature chip and thereby reduce mechanical and electrical artifacts.

The elimination of wires or cables between the patient and the medical instrument can be accomplished by the use of wireless or cable-less transducers. Wireless systems are known in the biomedical engineering field. For example, U.S. Patent No. 5,704,351 invented by Mortore discloses an eight-channel transmitter specifically directed to Electrocardiogram (ECG). This transmitter includes input circuitry, analog-to-digital converter and a microprocessor. It operates in the 902 to 928 MHz band, and it is adjustable by two manual setting switches. The use of these manual switches is the only adjustment available in the Mortare system for setting the carrier frequency. The input circuitry is not digitally controllable and thus this device cannot be adjusted by programming to be suitable for different biosignals with different characteristics. Furthermore, the Mortare device is only a conventional transmitter and does not contain RF receiver, which limits its applications.

U.S. Patent No. 6,755,230, issued to Schmidt et al. discloses a device for monitoring EEG. Similar to Mortare device, the Schmidt et al. device does not contain RF receiver and the transmitter is not programmable.

U.S. Patent No 5,579,775 issued to Dempsey discloses a patient's biotelemetry system.

Unlike Mortara and Schmidt et al., Dempsey includes an RF receiver which is used to control the system. However, the input circuitry is not adjustable by programming which limits the device applications.

U.S. Patent No. 6,167,258, entitled "Programmable Wireless Data Acquisition System" issued to Schmidt et al., discloses a programmable module with different inputs for signal processing of different signals and transmission to the base station. However, this system cannot be used with wireless (cable-less) transducers and it is not suitable to be used as a wireless mobile unit.

U.S. Patent No. 6,230,049, entitled "Integrated System for EEG monitoring and electrical stimulation with a Multiplicity of Electrodes", issued to Fischell et al., discloses a wireless link between the patient's EEG system and an EEG analysis workstation. Again the Fischell et al., module is limited only for EEG, and it does not contain wireless electrodes for EEG detection and the whole module cannot be used as a mobile unit.

U.S. Patent No. 6,026,321, entitled "System for measuring electrical potential variations in human body" issued to Miyate, et al., discloses a system for measuring the patients' biopotentials. The system includes electrodes, amplifier, voltage divider, compensator, a transmitter, and a battery that supplies required electric power to the amplifier and transmitter. The system acquires the potential variations from the electrodes through connecting wires. Further, the operation of the system is not software programmable, and thus not capable of accepting different input signal characteristics.

U.S. Patent No. 5,800,535, entitled "Wireless prosthetic electrode for the brain" issued to Howard, III, discloses a wireless neural prosthetic device that includes either a speech processor or a stimulating device and a wireless electrode. The device relates, in particular, to a wireless human central cortex neural prosthetic for delivering electrical signals to the patients primary auditory cortex. The Howard device is a therapeutic device, which is used to treat hard-of-hearing or deaf patient whose hearing cannot be restored with a cochlear implant.

U.S. Patent 5,862,803 and 6,708,050, entitled "Wireless electrode having activable power cell" both issued to Carim, discloses a galvanic power-cell suitable for biomedical wireless electrodes and electronic devices. It does not contain information regarding wireless electrodes.

U.S. Patent No 6,289,238 entitled "Wireless medical diagnosis and monitoring equipment" issued to Besson, et al., discloses a medical system for detecting EEG, ECG, and monitoring basic parameters such as temperature and perspiration. This system comprises wireless electrodes that include sensors or electrodes, sensor control, frequency generation, transmitting and receiving units, transmission control unit and antenna. However, the electrode does not include digitally programmable micro-power amplifiers and filters which limits the capability of the electrode for its use to measure different biosignals with different characteristics. Furthermore, the transceiver is a conventional transceiver and cannot be adjusted by programming which restricts its applications in multi-sensor or multi-electrodes measurements. Further it is not equipped with memory for data storage and does not provide primary analysis of captured biosignals and decisions.

The Besson monitoring system is not a mobile one. It is limited for measuring EEG and ECG with basic parameters such as temperature and perspiration. Besson failed to disclose the possible application of such system for measuring Parkinson disease, glucose, Phonocardiography, different types of apneas and thus this system lacks the expandability and ability to conduct multi-measurements. In addition the system does not provide self calibration. Another limitation of the Besson system is the lack of real time analysis of captured data by the system in order to give appropriate alerts on the spot based on advanced digital signal processing and intelligent algorithms. Furthermore, there is no mentioning of the common mode rejection ratio, equivalent input voltage and power consumption of the system.

U.S. Patents No., 5,307,818; 5,168,814 and 4,981,141, all issued to Segalowitz, discloses a wireless system for ECG monitoring. The Segalowitz patents describe a single-piece electrode patch with built-in microchips for wireless and one-way communication and a snap on electronic assembly that fastens to a disposable electrode patch. Electrodes can only be programmed via manual switches on electrode casing, not over-the-air (not wireless) from the base unit.

The Segalowitz patents further discloses a multiple electrode embodiments including a base unit containing multiple receivers and antennas, which imply multiple transmission frequencies, and thus making the base unit more expensive to implement. However, there is no mention of error correction or detection capability in the electrodes or base unit, and also no mention regarding the power consumption of this electrode. Thus, due to manual selection, the electrode is neither bi-directional nor programmable which limits its applications for different biomedical signals that have different characteristics. In addition the base unit is not mobile and lacks an alerting system and adaptive processing of the captured biosignals.

In another embodiment of the Segalowitz's '818 patent, there is a discussion of a single strip assembly which contains all of the electrodes required for 12-lead ECG monitoring with microchip circuitry contained in the strip assembly (not in the individual electrode patches). In this configuration, the ECG signals from each electrode are multiplexed and transmitted from a single transmitter contained in the strip assembly via time multiplexing on a single digitally encoded frequency channel. However, no time multiplexing on a single frequency channel is discussed for their multiple transmit electrode embodiment. Furthermore, the system is limited for ECG monitoring and the analysis of the captured ECG signals does not contain special adaptive procedures for the elimination of different artifacts such as: power line interference, electrode contact noise, motion artifact, muscle contraction and base line drift, instrumentation noise generated by electronic devices and electrosurgical noise. The connection between the electrodes with the microchip circuitry is made by wires, which implies that these electrodes cannot be used as a separated cable-less ones to transmit the captured ECG signals wirelessly from the electrodes to the monitoring system. Furthermore, it lacks of capability to transmit the biosignals wirelessly to the responsible physician or to remote server. Furthermore, the dimensions of the monitoring system are not small which means that it cannot be used as a mobile unit.

U.S. Patent No. 6,643,541, entitled "Wireless electromyography sensor and system" issued by Mok, et al., discloses a wireless module for measuring EMG. This module comprises wireless EMG electrodes and base station with no mobility or data analysis. Each wireless electrode comprises EMG electrode, analog-to-digital converter, transceiver, and micro controller computing platform. Thus it does not contain any adjustable or digitally programmable filters necessary to eliminate or reduce the DC offset of EMG electrodes or motion artifacts and interferences. In addition, the base station carries out the amplification, which is insufficient for the measurement of small-signal EMG signals in a noisy environment. Furthermore, the base station is not portable or mobile and it must be used as a bedside system, in addition, the proposed solution is limited for EMG. The CMRR and dimensions are other aspects that were not considered in Mok's patent. The wireless EMG electrode is not intelligent and is not able to realize the commands sent from the base station and to provide self- or auto-calibration.

The previous wireless systems described above are all limited for specific single application mainly to ECG, EEG, or EMG, with the ability to do one, but not multiple measurements. The electrodes are based on conventional techniques; they are not cable-less, intelligent, miniaturized, battery powered, programmable by software, bi-directional and capable of operating in different modes to extend battery life. None of the previous wireless systems are suitable for measuring Fetal ECG, phonocardiography, or glucose level nor are they suitable for conducting multiple measurements. Furthermore, the base station, which communicates wirelessly to the transducers connected to the patient, is not hand-held (portable) nor is it a mobile device. The prior art systems cannot provide remote information delivery and access that allow remote patients to be diagnosed by medical experts at remote locations. Moreover, they do not offer alert system that classifies the patient's health status based on intelligent algorithms. In addition to advanced analysis of the measured biosignals, no detailed information about the power consumption and dimensions of the wireless transducer, CMRR of the analog part, and total equivalent input noise voltage is available. These systems require external calibration and are not equipped with self- or auto-calibration procedures. Accordingly, the need remains for a mobile device based on modem communication technology that allows patient's biosignals collected by multiple cable-less transducers connected to a patient to be wirelessly transferred to another hand-held (portable mobile unit) which can transfer the biosignals to the mobile phone and then to the physician and remote central server unit.

### Summary of the invention

A mobile wireless system for capturing human biosignals via cable-less button-like transducers placed on human skin or implanted inside the human body of the patient. The mobile system includes at least four main parts: A. Intelligent miniaturized cable-less transducer, B. Wireless mobile unit, C. Computing device (Mobile phone handset, Personal Digital Assistant (PDA), or Personal Computer (PC)), and D. Internet/ Intranet servers. The system may be used to monitor different biopotentials (ECG, EEG, ERG, EMG, and EOG) and biosignals such as glucose, Phonocardiography and blood pressure. Captured information is presented to the physicians in appropriate medial presentation formats both numerically and graphically.

The transducer detects the biosignals and transmits them wirelessly (cable-less) to the mobile unit after suitable conditioning and processing, then the mobile unit provides the required digital signal processing of the received signal and displays or transmits it using Bluetooth or other transmission technology to the computing device which in turn may transmit the captured biosignal to the responsible physician or remote server unit in a hospital via centralized servers.

Complete elimination of wires between the transducer and the mobile unit minimizes the mechanical and electrical motion artifacts and greatly reduces the restriction on the patient's movement, which in turn improves the patient's comfort and compliance.

Geographically-remote (distant) information delivery and access allow rural or remote patients to be diagnosed by skillful physicians at any other remote locations (such medical care centers, home, work or even other rural location), in which without this invention it is required that the physician, the patient, and the capturing equipment has to exist in one physical location. The distant biosignal capturing can be conducted in on-line (real-time) and off-line fashions. The complete set of captured information (current and historical) can be accessed by one or more parties who can be located at many different geographical locations. All information captured is persisted in the patients' logs at centralized servers on the Internet and National Networks. The centralized servers offer direct human access interface, and Medical-standards-compliant protocol to access the information. In the former interface, the server software offers an alert system that uses color coding scheme to classify the problem nature. The alert system, which classifies the patient's status, is realized by novel intelligent algorithms developed using neuro-fuzzy approaches.

### Description of preferred embodiments

A more complete understanding of the method and system of the present invention may be acquired by reference to the following Detailed Description when taken in conjunction with the accompanying Drawings wherein:
- Fig. 1: illustrates an exemplary embodiment of the mobile system parts;
- Fig. 2: illustrates a block diagram of the cable-less transducer and the mobile unit within the mobile system;
- Fig. 3: illustrates a block diagram of the mobile system used for monitoring ECG and Phonocardiography;
- Fig. 4: illustrates a block diagram of the mobile system used for monitoring fetal ECG;
- Fig. 5: illustrates a block diagram of the mobile system used for monitoring ERG; and
- Fig. 6: illustrates a block diagram of the mobile system used for monitoring EEG.

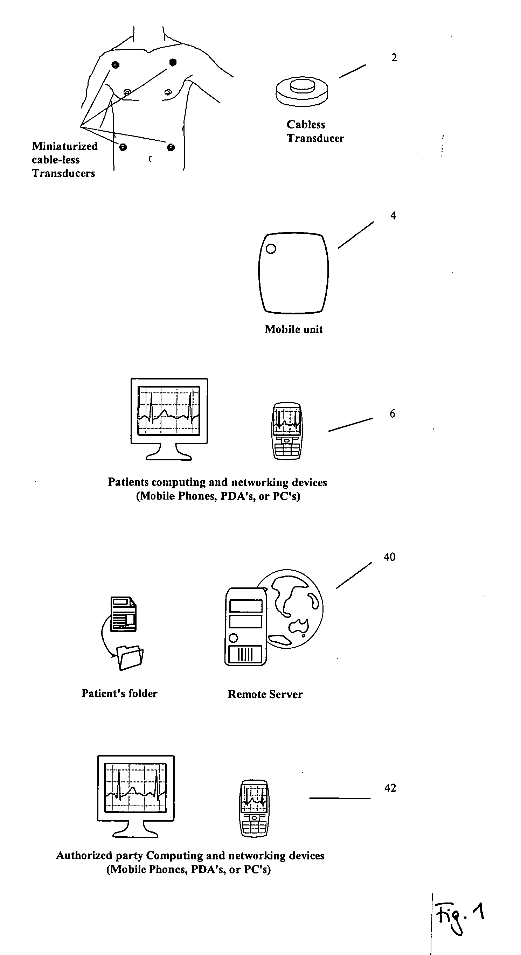

The present invention is a hand-held mobile system with a miniaturized cable-less intelligent transducer suitable for monitoring and analysis of different biomedical signals. The main parts of the system are shown in Figure 1 including a miniaturized intelligent cable-less transducer, a hand-held mobile unit, a computing device (mobile phone, PDA or PC), a remote server, patient files/folders, and access computing devices of the remote server. Each of these parts comprises the required blocks designed after taking into consideration all international standards for medical devices design and safety. The mobile system allows patients biosignals, collected by one or more miniaturized transducers connected to a patient to be transmitted - via wireless channel(s) - to a mobile unit worn by the patient or located in close proximity to the patient or at some distance. The transferred data might be displayed, analyzed, processed and transferred to the computing device which may also transmit the measured signal to the responsible physician or health center for further diagnoses and assistance. The wireless data transfer operates in both directions (bi-directional), that is, data can be also transferred from the miniaturized cable-less transducer connected to the patient to the mobile unit and then to the computing device and in the other direction from the computing device to the mobile unit and then to the miniaturized cable-less transducer connected to the patient.

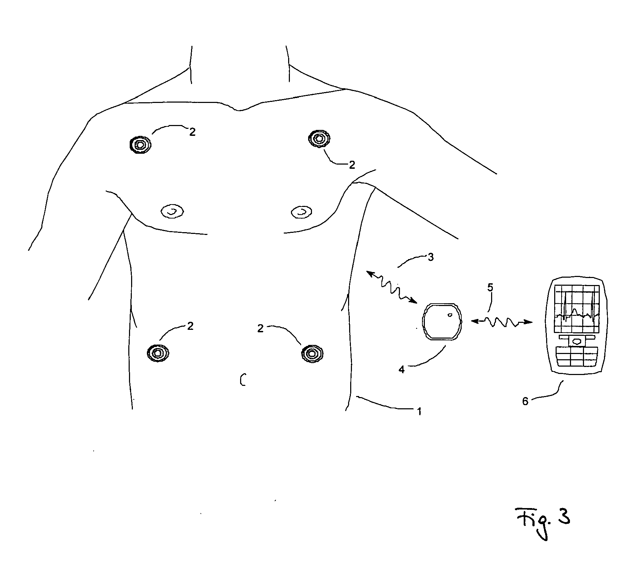

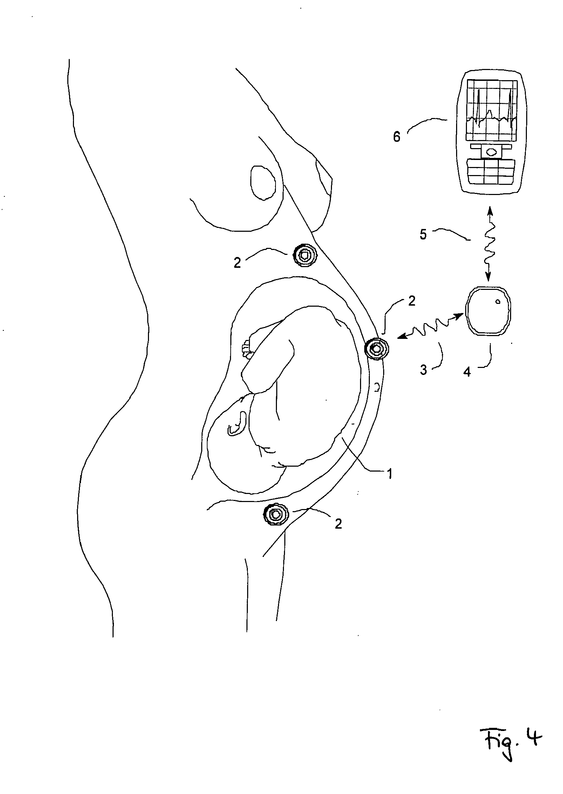

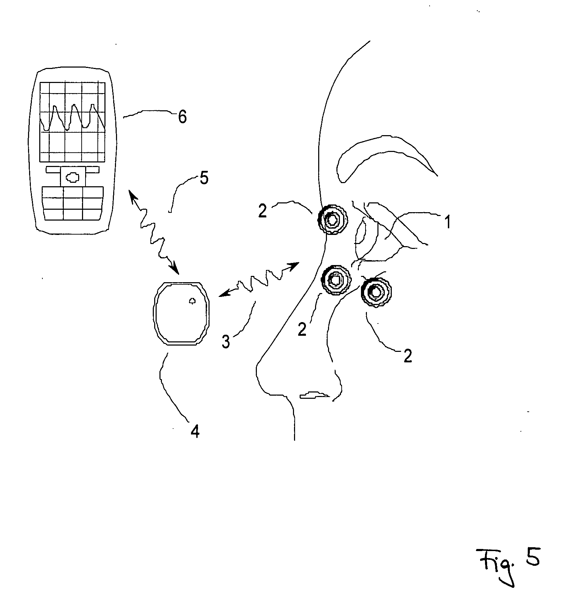

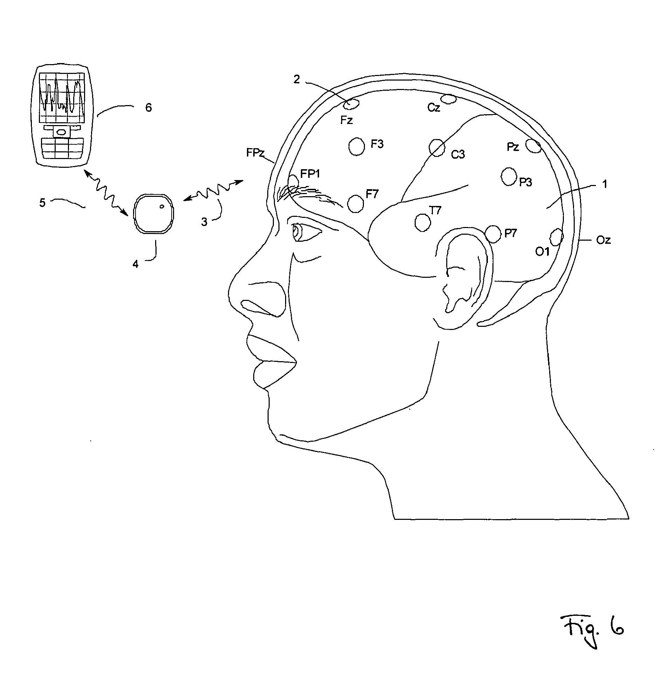

The system captures various types of human biosignals via cable-less button-like transducers that may be placed on the skin or implanted inside the body of the patient. The transducers may be placed as per the biosignal desired as described hereinafter with reference to figures 3, 4, 5, and 6 disclosing the ECG, fetal ECG, ERG, and EEG, respectively, are some of the applications on bio-potential sensing; Phonocardiography, respiration, temperature oxygen saturation, and blood glucose represent other examples (not shown in the figures). The applications are only limited by the available biosignal capturing (sensing) technologies.

As shown in Figure 2, there is illustrated the mobile system and its method of operation. The system includes a button-sized intelligent cable-less transducer **2** comprising the following main blocks; set of electrodes or sensors **8,** digitally programmable instrumentation amplifier **10,** digitally tunable active filters **12,** 12-bit analog-to-digital converter **14,** microcontroller **16,** programmable transceiver **21,** and miniaturized antenna **24.** All theses blocks except for the electrode or sensor are integrated into a single low power CMOS chip **25.** Thus it is powered using miniature long-life battery (not shown). The battery provides 3 V with a capacity of 1000 mA-H. The battery may be 12 mm long with a diameter of 7 mm. In an effort to extend battery life, three operating modes have been incorporated into the transducer design. These are a sleep or shutdown mode (current consumption <4 µA), a receiving mode (acquiring of biosignals and receiving commands from the mobile unit, current consumption <5 mA) and a transmit mode (largest current consumption <42 mA).

The first block in the cable-less transducer is the electrode or sensor **8.** There are two options, one for measuring biopotentials and the other for measuring biosignals. When a biopotential is measured an electrode is used, alternatively, when a biosignal which is not potential in its origin a sensor is used for such measurement. Thus, the sensor is used to convert the biosignals into electrical signals in order to provide required signals conditioning and processing.

The instrumentation amplifier **10** is a micro-power programmable amplifier used to amplify the measured biosignals. The voltage gain of this amplifier is adjustable by programming in the range of 1 to 10⁴. The digital control of this gain is realized by the microcontroller **16.** Thus, voltage gain is sufficient for amplification of different biosignals obtained from different electrodes and sensors **8.** To reduce the effect of common signals, the instrumentation amplifier **10** is designed with a 130 dB common mode rejection ratio (CMRR). Its sensitivity is less than 0.1 microvolt and the equivalent input noise voltage is less than 1 nV / sqrt (Hz). It is protected against defibrillator operation and its current consumption is less than 100 microamperes. The input stage of the amplifier is designed to ensure that the patients lead leakage current will always be less than 10 microamperes under any fault condition.

The active filter **12** is programmed to work as a 6^{th} -order low, high, or band pass filter with a cutoff frequency adjusted digitally by software to satisfy the desired application. The filter is primarily used to eliminate the DC offset resulting from the motion artifacts of biopotential electrodes and the high frequency noise and thus might act as an anti-aliasing filter. This filter is designed using micro-power devices with total current consumption less than 90 microamperes.

The output of the filter **12** is coupled to the input of high-resolution 12-bit analog-to-digital converter **14,** with a sampling rate ranges from 1000 samples per second up to 23,000 samples per second. The digital output of the A/D converter **14** is read by the high-performance and low-power microcontroller **16.** The microcontroller **16** has built-in 32 Kbytes of non-volatile flash memory and 2024 + 128 bytes of volatile random access memory and is able to operate in the sleep mode (non-operational) for reduced power consumption. The microcontroller **16** is further coupled to the programmable transceiver **21** to send the digital data to the programmable transceiver **21.** As shown in Figure 2, the microcontroller **16** is coupled to amplifier **10,** the filter **12** and the converter **14** to control all the components attached thereto.

The transceiver **21** has a programmable frequency in the range of 300 to 1000 MHz, high sensitivity programmable output power, very low current consumption and a single port antenna connection **23.** The antenna **24** used is a miniaturized one that has 10 mm length. Its is used to transmit the digital data (measured biosignal) wirelessly to the mobile unit **4** or receive commands from the mobile unit **4** such as transmission frequency commands, amplifier gain commands, filters cutoff frequency commands, transmitter control commands, and power saving mode commands. The received commands are realized by the microcontroller that includes the software required for processing these commands.

To avoid noises and interferences from other wireless systems that might be in the RF range and to organize the transmission of the number of transducers associated with the mobile unit **4,** time division multiplexing (TDM) is used that sets a specific time-slot per cable-less transducer or any other communication scheme may be used such as CDMA, etc. The mobile unit **4** receives the transmitted signal **3** from each transducer **2** at predetermined signal time slot using miniaturized antenna **26** in the mobile unit **4.** The antenna **26** is coupled to the bi-directional transceiver **28** which in turn is programmable and set to correspond to the frequencies of the transducers **2.** The transceiver **28** is coupled to a microcontroller **30** having a digital signal processing unit primarily used to control the operation of the mobile unit **4.** The microcontroller **30** may further display the control operations on a display unit **33** (e.g., LCD display). The microcontroller **30** further after digitally processing the data (after proper error detection and correction) and applying the needed filtration of the signals, sends the processed signals to a transmission unit **34** (e.g., Bluetooth) that may send the desired signals using transmission antennas **35,** wirelessly, to the mobile computing device **6** (smart mobile phone, digital personal assistant or personal computer).

According to the present invention, the mobile unit **4** identifies available cable-less transducers, receives captured biosignal from the cable-less transducers, conduct further signal analysis (DSP), transmits biosignals to a computing device using proximity wireless standard such as Bluetooth, and receives commands from computing device to select input channels, and issue control actions to the transducers.

Digital RF signal is transmitted from the transducers **2** to the mobile unit **4** which in turn controls the operation of all transducers and transmits the signal to a generic computing device **6** after providing wavelet analysis and adaptive filtration of received biosignals.

Hence the user can preview the captured biosignal data in both graphical and numeric forms on that computational device **6** such as smart mobile phones, personal digital assistant, and personal computers and then to be transmitted to medical personnel for advice or consultation or to persist the data on the remote central server.

The design of the transducer **2** is made with optimized power consumption and optimized size. Signal captured is transmitted to proximity mobile unit **4** that is carried-on by the patient or placed in a near-by position.

The mobile unit **4** orchestrates the operation of the different transducers and manages them in order to generate the readings as instructed by the physician. The mobile unit **4** is controlled via a computing device **6** providing User Interface (UI) to select biosignal channels, and to graphically or textually view the results. The computing device **6** can transmit the signal via available telecommunication infrastructure to the remote central server **40** on the Internet. The received biosignals along with related information is placed and used to update the patients folder. The remote central server **40** conducts analysis and basic diagnosis in order to arrive at the severity level, using color coding, described below, and can generate alerts to interested parties such as physicians or medical centers. The status alert generated may be an alert in several possible forms such as Email, SMS, phone call, etc. Upon that alert or at any other time, the physician or other authorized parties, can access captured records (i.e., biosignals and history) on the server **40** and view them even remotely using, again, available telecommunications infrastructure via an access computing device **42.**

The remote central server shall hold all that information providing additional capabilities to make graphs, diagnosis or generate commands via programmed flow or manually controlled. Data is persisted on the server in DICOM compatible format. Alerts are generated to interested parties in a color-coded scheme.

The different system units possess digital logic and programming intelligence allowing for automatic-reflex controllability and automatic signal conditioning and analysis which helps in making immediate actions, either locally, or through alerting the physician or medical center.

Remote central unit alert and escalation is based on the analysis of the biosignals and the results are presented in a convenient color-coding system. This is carried out by the intelligent program developed using neuro-fuzzy approaches after wavelet analysis and adaptive processing of captured biosignals. The color coding system is divided into four levels:
RED - LEVEL 4: life-threatening status. Immediate action is necessary in less than 10 minutes. Examples of these cases are Ventricle Tachycardia (VT), hyperacute T-wave (MI or high K).
ORANGE - LEVEL 3: critical status that requires medical attention. Examples of these cases are Atrial fibrillation (AF), Atrial Holter < 110, sinus tachycardia rate > 120.
YELLOW - LEVEL 2: abnormal status, which requires medical checkup.
WHITE - LEVEL 1: Normal status.

The patient could also do direct reporting of his or her felt symptoms for example: shortness of breath, chest pain, jaw pain, shoulders pain, hands pain, epegastric pain. All those are reported as red states, upon that the physician can instruct to have all the possible and needed biosignal readings such as blood-pressure, respiration, temperature, oxygen saturation, alongside the ECG. The advantages of such a system are numerous, spanning the spectrum of human medical welfare, health industry, government organizations, reducing deployment care, decreasing wiring cost and providing safer care to patients.

According to the present invention, the system captures various types of biosignals via the cable-less button-like transducers that may be placed on the skin or implanted inside the body of the patient.

Figure 3 illustrates the used of the mobile system to measure ECG biopotentials according to an embodiment of the present invention. The positions of the transducers **2** placed on the body of the patient are as shown in the figure. However, it should be understood that the positions of each transducer may be different from what is shown with no departure from the teachings of the present invention.

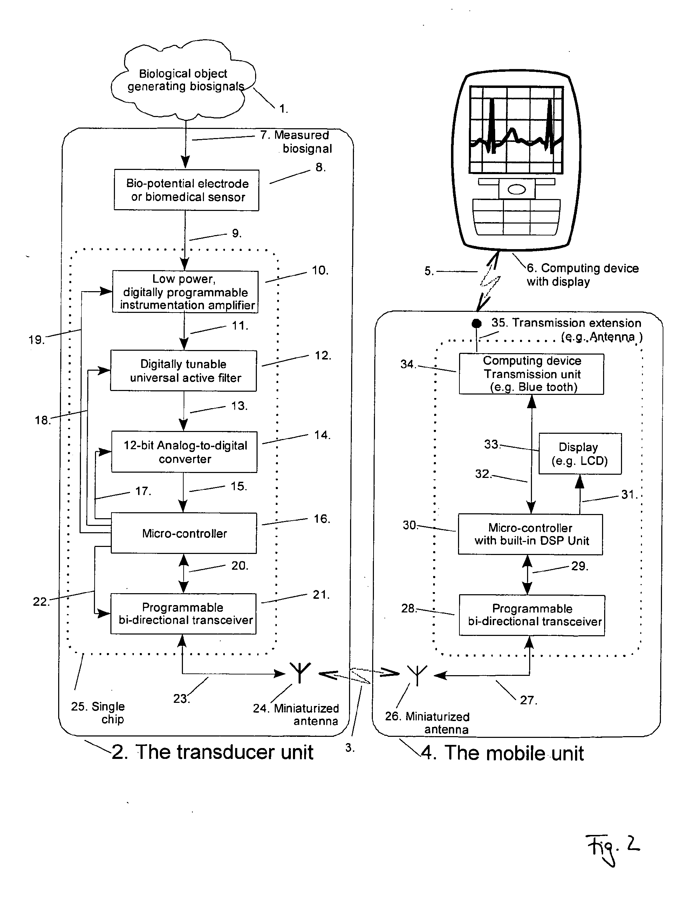

Similarly, Figures 4, 5, and 6 each disclose the use of the mobile system in measuring fetal ECG, ERG, and EEG, respectively, according to preferred embodiments of the present invention. The positions of the transducers **2** placed on the body of the patient are as shown in the figures. However, it should be understood that the positions of each transducer may be different from what is shown with no departure from the teachings of the present invention.

In a preferred embodiment of the present invention, the mobile system may be used to monitor and analyze different biosignals with the capability of providing multi measurements, which ensure accurate diagnosis of investigated patient. For example, but not limited to, the system is capable of measuring simultaneously 12-lead ECG, phonocardiography, heart rate and heart rate variability. This gives the physician comprehensive data and enables him or her to take more correct diagnostic decision. Again, the system can also perform different types of simultaneous measurements of biomedical signals such as ECG together with EEG and EMG. The software part designed for the mobile unit is written to optimize the processing and analysis for various inputs from the cable-less transducers, and to act as an orchestrate for them. The software part designed for the computing device allows the user to select types of inputs to view and graph and send commands to the cable-less transducers **2** (via the mobile unit **4).** The computing device **6** software also allows the user to send or retrieve the captured information into the DICOM compatible remote central server unit **40.** This is achieved due to the use of fully digitally programmable cable-less transducer **2** and mobile unit **4** with the ability of conducting advanced digital signal processing to the captured biosignals.

In another preferred embodiment of the present invention, the system is capable of capturing new important biosignals not measured before by wireless methods. Those parameters are: Blood glucose level, Fetal ECG and phonocardiography. The wireless measurement of the glucose level is made possible due to the design of a wireless glucose sensor. The development of such transducer will not only allow wireless measurement of such as blood glucose but also develop significantly the closed-loop artificial organs such as the artificial pancreas. Those skilled in the art will realize that the mobile system for monitoring Glucose level, Fetal ECG, and phonocardiography using cable-less transducers was not possible with conventional techniques.

In another preferred embodiment of the present invention, the biosignals are collected from the patient using a bi-directional, cable-less, miniaturized, battery-powered and intelligent transducer **2;** the transducer **2** might be cable-less electrode if a biopotential is desired to be measured, or a cable-less sensor if the original biosignal is not potential, such as glucose level. The dimension of this transducer may be about 15 mm radius by 5mm thickness and the current consumption may be about a few milli amperes (powered by a miniature battery). The transducer **2** according to the present invention is capable of providing programmable voltage gain, programmable universal active filter, auto-calibration and digitally tunable transmission frequency. The use of cable-less transducer eliminates completely the need for wires between the transducers and the mobile unit which reduces the motion artifacts and improves the patient's movement, comfort and compliance.

In another preferred embodiment of the present invention, the mobile unit **4** is a small hand-held, battery powered and fully programmable device. It may weighs less than 200 grams, with dimensions (length x width x height) 70 mm x 40 mm x 30 mm) and has low power consumption. It includes as described hereinabove, a programmable transceiver **28,** microcontroller with built in Digital Signal Processor (DSP) unit **30,** and computing device transmission unit **34** (Bluetooth). The mobile unit **4** receives the transmitted signals from the cable-less transducer **2** and performs many tasks that greatly simplify the use and implementation of the mobile system. Those tasks involve: error detection and correction, digital signal processing (wavelet transform and adaptive filtration), bi- directional transmission of biosignals, sending control commands to the cable-less transducer and classification of patient's health status using intelligent algorithms. Thus, this mobile unit can be worn by the patient without affecting his or her comfort or restricting his or her movement, which is very necessary especially in case of its use as a Holter monitor.

In another preferred embodiment of the present invention, the measured biosignal can be presented on a wide range of computing devices with wireless connectivity hardware such as Bluetooth. Examples of the computing devices are PDA (Such as Linux, Windows CE, and PalmOS based PDAs), PC's with Windows or Linux operating systems, and Mobile phones with Symbian OS platform. The computing device software part is designed in such as way to run on most types of smart mobile phones, personal digital assistant (PDA), and personal computers. Providing rich graphical interface that allows the user to easily view captured data, diagnosis results, and store or retrieve from the remote central server. The mobile phone 6 receives the biosignal transmitted from the mobile unit **4** via Bluetooth and monitors or transmits it wirelessly to the physician or health care center.

In another preferred embodiment of the present invention, the mobile system provides primary diagnosis and thus health advice for the patient using four level-color coding schemes. Red means life threatening status, Orange means critical status, Yellow means abnormal, and White means normal status. Of course this is very important especially for critically ill patients such as heart patients. Furthermore, to improve the diagnosis accuracy of the patient's health status, the system alerts are generated based on intelligent algorithms realized using neuro-fuzzy approaches.

In another preferred embodiment of the present invention, the mobile system provides a wide range of applications that enable large groups of patients to benefit from the system. Examples of system applications are: assistant in case of accidents and emergencies, early diagnosis and reporting of issues, lifetime patient history records, improvement of health standard of living with lower cost for hospitals, insurance companies, and health-care government agencies, assistance and monitoring in home-care settings, monitoring of chronically ill patients, patient involvement in setting diagnosis, medicine dosage adjustment, physical status monitoring in sports, monitoring for irregular symptoms that are manifested at unexpected times and emergency alarms especially in intensive care units. Examples (not limited to) of large groups of patients that can benefit from this system are:
- Heart condition patients: Heart parameters like phonocardiography, heart rate, ECG, and blood pressure can be simultaneously monitored, that aid physicians in diagnosis and treatment of heart patients.
- Asthma/respiratory patients: parameters like oxygen levels and respiratory rate can be closely monitored to aid physicians in setting diagnosis.
- Diabetes patients: by monitoring blood glucose levels or insulin levels, physicians and patients can be aided in diagnosis and treatment of diabetes and its symptoms.
- Biopotential measurements such as ECG, EMG, EEG, EOG and ERG, which enable the physicians to diagnose critical patient diseases such as biopsy and Parkinson disease.

The present invention was made possible due to the use of intelligent cable-less transducers suitable for desired biosignals and the capability of the system to be programmed to measure different types of biosignals.

Although preferred embodiments of the method and system of the present invention have been illustrated in the accompanying Drawings and described in the foregoing Detailed Description, it will be understood that the invention is not limited to the embodiments disclosed, but is capable of numerous rearrangements, modifications and substitutions without departing from the spirit of the invention as set forth and defined by the following claims.

## Claims

1. A mobile system for monitoring and analyzing different biosignals comprising: at least one intelligent miniaturized cable-less transducer, a mobile unit coupled via a wireless connection to said transducer and a computing device coupled to said mobile unit for receiving signals from said mobile unit and analyzing said signals to generate data, said generated data being transmitted by said computing device to a remote server via an available communication channel.

2. The mobile system of claim 1, wherein said biosignals comprise ECG biopotential, EEG biopotential, ERG biopotential, EMG biopotential, Fetal ECG biopotential, glucose level biosignal, or respiratory parameters biosignal.

3. The mobile system of claim 1 or 2, wherein said intelligent cable-less transducer eliminates the need for wires to transmit the captured biosignals to the mobile unit, thereby reducing the motion artifacts and improves the patient's mobility, comfort and compliance; wherein said cable-less transducer is battery powered, miniaturized, bi-directional, programmable by software, and designed to operate in a number of modes to extend battery life.

4. The mobile system of one of the previous claims, wherein said cable-less transducer comprises a sensor to convert the biosignals into an electrical signal and an electrode to acquire the biopotential.

5. The mobile system of claim 4, wherein said cable-less transducer comprises a micro-power programmable amplifier connected to the output of said sensor or to said electrode for amplification of the output signal of said sensor or electrode.

6. The mobile system of claim 5, wherein said cable-less transducer comprises:
a micro-powered digitally programmable universal active filter coupled to the output of said amplifier to eliminate DC offset resulting from motion artifacts of electrodes during biopotential measurement or from high frequency noise, wherein the cut-off frequency and type of said active filter is digitally programmable; and
a 12-bit analog-to-digital converter coupled to the output of said programmable active filter.

7. The mobile system of one of the previous claims, wherein said cable-less transducer comprises a microcontroller, said microcontroller comprises Random Access Memory (RAM) to store measured data and Electrical Programmable Random Access Memory (EPROM) to store a program that controls the operation of all parts of the cable-less transducer.

8. The mobile system of claim 7, wherein said microcontroller controls the voltage gain of said amplifier, the cut-off frequency and type of the active filter, the operation of A/D converter, the transmitting and receiving frequencies of the transceiver, the communication with the mobile computing device, preliminary analysis of measure biosignals, data storage, data transmission to said mobile computing device and realizing the receiving commands from the mobile unit and providing auto-calibration for the used transducers.

9. The mobile system of one of the previous claims, wherein said cable-less transducer comprises:
a digitally programmable bi-directional transceiver; and
miniaturized antenna coupled to said transceiver to transmit or receive digital signals to or from the mobile unit.

10. The mobile system of one of the previous claims, wherein said intelligent cable-less transducer being powered by miniature battery and designed to operate in three modes (to extend battery life), wherein said three modes comprises sleep or shutdown mode, a receiving mode and a transmit mode.

11. The mobile system of one of the previous claims, wherein said intelligent cable-less transducer is a cable-less biopotential electrode or a cable-less sensor for wirelessly measuring different biosignals including blood glucose level.

12. The mobile system of one of the previous claims, wherein said the intelligent cable-less transducer is based on time-division multiplexing transmission, thereby avoiding interference from other wireless system.

13. The mobile system of one of the previous claims, wherein said intelligent cable-less transducer is designed as a safe miniaturized single chip transducer that can be placed on human skin or implantable inside the human body.

14. The mobile system of one of the previous claims, wherein said mobile unit comprises a microcontroller with built in Digital Signal Processing (DSP) unit, bidirectional transceiver and a computing device transmission system, wherein said DSP unit provides wavelet analysis and adaptive digital filtration of captured biosignals to eliminate the noise from the captured biosignals.

15. The mobile system of claim 14, wherein said bidirectional transceiver controls the wireless communication between the cable-less transducer and the mobile unit and said transmission system controls the wireless communication between the mobile unit and a computing device.

16. The mobile system of one of the previous claims, wherein said mobile system is capable of monitoring and diagnosis the patient's health status whiles both the patient and physician are geographically separated, said mobile system utilizes existing wireless infrastructures comprising GSM, CDMA, Satellite mobile phones, Modems, or ADSL land phones, thereby, allowing freedom of mobility with geographical distance.

17. The mobile system of one of the previous claims, wherein said mobile system is capable of providing alert procedure with escalation using intelligent neuro-fuzzy approach based on more rigid analysis conducted by powerful hardware at said remote server, said mobile system triggers alerts by phone or SMS, thereby enabling the medical staff to be aware of the severity of the case using an easy to comprehend color coding scheme, wherein data is available to physicians independent of the monitoring device and for any historical references to authorized parties including medical centers and physicians, wherein said remote server maintains DICOM compatible access interface, thereby, providing persistence and availability of said data.

18. A method for measuring biosignals comprising the following steps:
measuring biosignals using at least one miniaturized intelligent transducer connected to a patient;
transmitting said biosignals via a wireless connection to a mobile unit; and
analyzing said biosignals via a computing device.

19. The method according to claim 18, further comprising the step of:
managing the operation of said at least one miniaturized intelligent transducer, wherein said measuring step comprises measuring a plurality of different biosignals using a plurality of said at least one miniaturized intelligent transducer.

20. The method according to claim 18 or 19, further comprising the steps of:
controlling the voltage gain of an amplifier, the cut-off frequency and type of an active filter, and the operation of an A/D converter; and
analyzing said measured biosignals.
